(19) Europäisches Patentamt
European Patent Office
Office européen des brevets

(11) EP 4 541 332 A1

(12) EUROPEAN PATENT APPLICATION
published in accordance with Art. 153(4) EPC

(43) Date of publication:
23.04.2025 Bulletin 2025/17

(51) International Patent Classification (IPC):
A61F 13/42 (2006.01)    A61F 13/511 (2006.01)
A61F 13/537 (2006.01)

(21) Application number: 23845923.4

(22) Date of filing: 13.04.2023

(52) Cooperative Patent Classification (CPC):
A61F 13/15; A61F 13/42; A61F 13/47;
A61F 13/472; A61F 13/475; A61F 13/511;
A61F 13/513; A61F 13/533; A61F 13/537

(86) International application number:
PCT/JP2023/015043

(87) International publication number:
WO 2024/024183 (01.02.2024 Gazette 2024/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.07.2022 JP 2022120400

(71) Applicant: Unicharm Corporation
Shikokuchuo-shi, Ehime 799-0111 (JP)

(72) Inventors:
• HASHINO, Akira
  Kanonji-shi, Kagawa 769-1602 (JP)
• NOMOTO, Takashi
  Kanonji-shi, Kagawa 769-1602 (JP)
• SUZUKI, Yuya
  Kanonji-shi, Kagawa 769-1602 (JP)
• HAYASHI, Toshihisa
  Kanonji-shi, Kagawa 769-1602 (JP)

(74) Representative: Dolleymores
9 Rickmansworth Road
Watford, Hertfordshire WD18 0JU (GB)

(54) ABSORBENT ARTICLE

(57) An absorbent article in which it is possible to easily maintain absorbing capabilities of an absorbent core, while liquid is prevented from remaining on the topsheet is provided. An absorbent article (1) includes a liquid-permeable topsheet(10) , a liquid-impermeable backsheet(20) , and an absorbent core (31) positioned between the topsheet and the backsheet. A low liquid permeation portion (100) having a lower permeability speed for body fluid to permeate from a skin side to a non-skin side than surroundings thereof is provided on a non-skin side relative to a non-skin surface of the topsheet and on a skin side relative to a non-skin surface of the absorbent core. The low liquid permeation portion is placed in a central region (RC) positioned in a central part in a width direction among regions obtained by equally dividing the absorbent core into three regions in the width direction.

FIG. 3

## Description

[TECHNICAL FIELD]

The present invention relates to an absorbent article.

[Background Art]

Patent Literature 1 discloses an absorbent article including a low liquid permeation portion. The low liquid permeation portion in Patent Literature 1 is provided on a topsheet. The topsheet is obtained by applying a liquid repellent to a spunlace non-woven fabric including 100% by weight of cotton fibers, while a large number of pores penetrating from one surface to the other are formed in at least a portion corresponding to an excretion opening, in an oblong formation that is long in the lengthwise direction of the absorbent article. The region to which the liquid repellent has been applied has lower liquid absorbency on a skin contact surface and has thus a lower capability to absorb body fluid than surroundings thereof. Further, by guiding body fluid to a non-skin side through the pores, it is possible to prevent liquid from remaining on the topsheet.

[Citation List]

[Patent Literature]

[Patent Literature 1] Japanese Patent Laid-Open No. 2017-99558

[Summary of Invention]

However, the absorbent article described above has the following problems:
As for the body fluid discharged on the topsheet, a certain part of the body fluid that has been guided to the non-skin side through the pores does not remain on the topsheet. It is therefore possible to prevent liquid from remaining on the topsheet. However, some body fluid positioned in the region having the liquid repellent applied thereto is not easily drawn into the topsheet. Such body fluid thus easily remains on the topsheet. As a result, there is a possibility that the body fluid remaining on the topsheet may cause skin trouble.

In addition, the body fluid drawn in via the pores in the topsheet is guided to an absorbent core and held by the absorbent core. The applicant conducted intensive study and learned that, as a result of absorbing body fluid, rigidity and thickness of the absorbent core change. As compared to the absorbent core before the absorption, the absorbent core that has absorbed the body fluid may be unable to maintain the rigidity and the thickness thereof, and therefore may be unable to maintain a goal state until after use.

The present invention has been made in view of the problems described above, and an object thereof is to provide an absorbent article in which it is possible to easily maintain absorbing capabilities of an absorbent core, while liquid is prevented from remaining on the topsheet.

An absorbent article according to an Aspect includes a liquid-permeable topsheet, a liquid-impermeable backsheet, and an absorbent core positioned between the topsheet and the backsheet. A low liquid permeation portion having a lower permeability speed for body fluid to permeate from a skin side to a non-skin side than surroundings thereof is provided on a non-skin side relative to a non-skin surface of the topsheet and on a skin side relative to a non-skin surface of the absorbent core. The low liquid permeation portion is placed in a central region positioned in a central part in a width direction among regions obtained by equally dividing the absorbent core into three regions in the width direction.

An absorbent article according to another Aspect includes a liquid-permeable topsheet, a liquid-impermeable backsheet, and an absorbent core positioned between the topsheet and the backsheet. A low liquid permeation portion having a lower permeability speed for body fluid to permeate from a skin side to a non-skin side than surroundings thereof is provided on a non-skin side relative to a non-skin surface of the topsheet and on a skin side relative to a non-skin surface of the absorbent core. The low liquid permeation portion is provided on the absorbent core. An absorption amount per unit area of the absorbent core provided with the low liquid permeation portion is smaller than an absorption amount per unit area of the absorbent core positioned in the surroundings of the low liquid permeation portion. Rigidity of a region provided with the low liquid permeation portion is higher than rigidity of a region in the surroundings of the low liquid permeation portion.

[Brief Description of Drawings]

[FIG. 1] FIG. 1 is a plan view of an absorbent article according to an embodiment as seen from a skin side.

[FIG. 2] FIG. 2 is a plan view of the absorbent article according to the embodiment as seen from a non-skin side.

[FIG. 3] FIG. 3 is a cross-sectional view taken along the line A-A indicated in FIG. 1.

[FIG. 4] FIG. 4 is a cross-sectional view taken along the line B-B indicated in FIG. 1.

[FIG. 5] FIG. 5 is a drawing showing an absorbent article according to modification examples.

[DESCRIPTION OF EMBODIMENTS]

(1) Outline of Embodiments

The description will be given at least about the following points with reference to the specification and the accompanying drawings.

An absorbent article according to Aspect 1 includes a liquid-permeable topsheet, a liquid-impermeable backsheet, and an absorbent core positioned between the topsheet and the backsheet. A low liquid permeation portion having a lower permeability speed for body fluid to permeate from a skin side to a non-skin side than surroundings thereof is provided on a non-skin side relative to a non-skin surface of the topsheet and on a skin side relative to a non-skin surface of the absorbent core. The low liquid permeation portion is placed in a central region positioned in a central part in a width direction among regions obtained by equally dividing the absorbent core into three regions in the width direction. The low liquid permeation portion is positioned on the skin side relative to the non-skin surface of the absorbent core. As for the absorbent core, to a certain part of the absorbent core positioned on the non-skin side relative to the low liquid permeation portion , the body fluid is guided less easily, which makes the absorption amount of body fluid smaller. Usually, the rigidity and the thickness of the absorbent core change when body fluid is absorbed. As compared to the absorbent core before the absorption, the absorbent core that has absorbed the body fluid might be unable to maintain the rigidity and the thickness thereof, and therefore might be unable to maintain a goal state until after use. However, by providing the low liquid permeation portion, it is possible to provide the absorbent core with a portion where it is possible to prevent changes in the absorbent core that may occur between before and after the body fluid absorption. In the central region of the absorbent core, because the body fluid is more easily discharged therein, a larger change is to be caused by the body fluid absorption. Providing the central region with the low liquid permeation portion makes it easier to maintain, even after use, the state prior to use. Further, it is possible to ensure drawability of the body fluid for the topsheet and the like positioned on the skin side relative to the low liquid permeation portion and to thus prevent liquid from remaining on the surface of the absorbent article . Consequently, it is possible to prevent skin trouble that may be caused by the body fluid remaining on the surface of the absorbent article.

According to a preferred aspect, the invention according to Aspect 2 may have the following features in the invention according to Aspect 1. The rigidity of the region provided with the low liquid permeation portion is higher than the rigidity of the surroundings regions of the low liquid permeation portion. Because the rigidity of the region provided with the low liquid permeation portion is higher than that of the surroundings, there is a lower possibility that deformation may be caused by pressure from the body during use, and the state prior to use can therefore be maintained more easily. Furthermore, because of being positioned on the non-skin side relative to the topsheet , the low liquid permeation portion does not easily touch the skin of the user directly. Because the rigidity of the topsheet is lower than the rigidity of the low liquid permeation portion , even when the rigidity of the low liquid permeation portion itself is high, the user does not easily feel high rigidity parts because of softness of the topsheet. It is therefore possible to prevent discomfort during use.

An absorbent article according to Aspect 3 includes a liquid-permeable topsheet, a liquid-impermeable backsheet, and an absorbent core positioned between the topsheet and the backsheet. A low liquid permeation portion having a lower permeability speed for body fluid to permeate from a skin side to a non-skin side than surroundings thereof is provided on a non-skin side relative to a non-skin surface of the topsheet and on a skin side relative to a non-skin surface of the absorbent core. The low liquid permeation portion is provided on the absorbent core. An absorption amount per unit area of the absorbent core provided with the low liquid permeation portion is smaller than an absorption amount per unit area of the absorbent core positioned in the surroundings of the low liquid permeation portion. Rigidity of a region provided with the low liquid permeation portion is higher than rigidity of a region in the surroundings of the low liquid permeation portion. Because the absorption amount per unit area of the region provided with the low liquid permeation portion is lower, it is possible, by providing the low liquid permeation portion, to provide the absorbent core with the part where it is possible to prevent the changes in the absorbent core that may occur between before and after the body fluid absorption. In addition, because the rigidity of the region provided with the low liquid permeation portion is higher than that of the surroundings, there is a lower possibility that deformation may be caused by pressure from the body during use, and the state prior to use can therefore be maintained more easily. Furthermore, because of being positioned on the non-skin side relative to the topsheet ,the low liquid permeation portion does not easily touch the skin of the user directly. Because the rigidity of the topsheet is lower than the rigidity of the low liquid permeation portion , even when the rigidity of the low liquid permeation portion itself is high, the user does not easily feel high rigidity parts because of softness of the topsheet. It is therefore possible to prevent discomfort during use.

According to a preferable aspect, the invention according to Aspect 4 may have the following features in the invention according to any one of Aspects 1 to 3. The low liquid permeation portion is made of a liquid-impermeable sheet positioned on the skin side relative to the absorbent core. According to the present aspect, the liquid-impermeable sheet prevents the body fluid from being absorbed from the skin side. It is therefore possible to ensure the rigidity of the absorbent core in the region overlapping with the sheet.

According to a preferable aspect, the invention according to Aspect 5 may have the following features in the invention according to any one of Aspects 1 to 3. The low liquid permeation portion is made of a hydrophobic coating agent applied to at least one of the absorbent core, the topsheet, and a sheet positioned between the absorbent core and the topsheet. According to the present aspect, the hydrophobic coating agent prevents the body fluid from being absorbed from the skin side, and it is possible to ensure the rigidity of the absorbent core in the region overlapping with the adhesive agent.

According to a preferable aspect, the invention according to Aspect 6 may have the following features in the invention according to any one of Aspects 1 to 3. The low liquid permeation portion is made of a compressed part obtained by compressing at least the absorbent core in a thickness direction. According to the present aspect, because the low liquid permeation portion including the compressed part has high rigidity, it is possible to further ensure the rigidity of the absorbent core .

According to a preferable aspect, the invention according to Aspect 7 may have the following features in the invention according to any one of Aspects 1 to 3. The low liquid permeation portion is provided between the topsheet and the absorbent core. According to the present aspect, it is possible to prevent body fluid absorption of the absorbent core in at least the region overlapping with the low liquid permeation portion . In addition, because the low liquid permeation portion and the absorbent core are provided separately from each other, it is possible to easily ensure the absorbing capabilities of the absorbent core.

According to a preferable aspect, the invention according to Aspect 8 may have the following features in the invention according to any one of Aspects 1 to 3. The low liquid permeation portion is provided on the absorbent core. According to the present aspect, due to the presence of the low liquid permeation portion, the absorption does not easily occur in the absorbent core, and it is therefore possible to maintain rigidity of the absorbent core.

According to a preferable aspect, the invention according to Aspect 9 may have the following features in the invention according to any one of Aspects 1 to 3. The absorbent article includes a second sheet positioned between the topsheet and the absorbent core. The low liquid permeation portion is provided between the topsheet and the second sheet. According to the present aspect, it is possible to lower the possibility of the absorbent core absorbing body fluid and to thus further decrease the changes in the rigidity of the absorbent core that may occur between before and after the body fluid absorption.

According to a preferable aspect, the invention according to Aspect 10 may have the following features in the invention according to any one of Aspects 1 to 9. The low liquid permeation portion straddles a center, in the width direction, of the absorbent core. The center, in the width direction, of the absorbent core is a portion where force gets easily concentrated, when interposed between the legs or the like. In addition, the center, in the width direction, of the absorbent core is a region where body fluid is easily discharged. By providing the low liquid permeation portion so as to straddle the center, in the width direction , of the absorbent core, it is possible to prevent deformation caused by such force and also to prevent the changes in the rigidity and the like that may be caused by the absorption of the body fluid.

According to a preferable aspect, the invention according to Aspect 11 may have the following features in the invention according to any one of Aspects 1 to 10. The low liquid permeation portion extends along a front-rear direction. According to the present aspect, with the low liquid permeation portion , it is possible to spread the body fluid along the front-rear direction and to absorb the body fluid in a large region extending along the front-rear direction. By having the larger range for absorbing the body fluid, it is possible to avoid locally forming a region where a large amount of body fluid is held and to thus easily maintain the rigidity and the like of the absorbent core even after the absorption of the body fluid.

According to a preferable aspect, the invention according to Aspect 12 may have the following features in the invention according to Aspect 11. A plurality of low liquid permeation portion are provided at intervals in the width direction. According to the present aspect, the low liquid permeation portions are positioned at intervals in the width direction , so that a region having no low liquid permeation portion is present between the low liquid permeation portions. Thus, even when the low liquid permeation portion positioned on the outer side in the width direction is deformed due to force being applied by the legs or the like inwardly in the width direction , the low liquid permeation portion positioned on the inner side in the width direction does not get deformed and thus exerts capabilities thereof. As a result, one of the low liquid permeation portions is able to maintain the capabilities of the low liquid permeation portions.

According to a preferable aspect, the invention according to Aspect 13 may have the following features in the invention according to any one of Aspects 1 to 10. A plurality of low liquid permeation portion extends along the width direction and are provided at intervals in a front-rear direction. According to the present aspect, the low liquid permeation portions are positioned at intervals in the front-rear direction so that regions having no low liquid permeation portion are present between the low liquid permeation portions. When the absorbent article is positioned along a curve of the body from the front side to the rear side of the body, the low liquid permeation portions are able to exert capabilities thereof without being deformed, and at least one of the low liquid permeation portions is able to maintain the capabilities of the low liquid permeation portion Further, while body fluid is being drawn to the non-skin side in the region positioned between the low liquid permeation portions, the low liquid permeation portions are able to spread the body fluid in the width direction, and it is therefore possible to absorb the body fluid in a large region extending along the width direction. Further, in a mode in which the low liquid permeation portion has high rigidity, because the low liquid permeation portion is able to resist force from the legs, it is possible to prevent excessive deformation of the absorbent core.

According to a preferable aspect, the invention according to Aspect 14 may have the following features in the invention according to any one of Aspects 1 to 13. A dimension of the low liquid permeation portion in the width direction is equal to or smaller than 50% of a dimension of the absorbent core in the width direction. According to the present aspect, the dimension of the low liquid permeation portion in the width direction is equal to or smaller than 50% of the entire span of the absorbent core in the width direction. Thus, the low liquid permeation portion does not easily touch the legs or the like of the user and is thus able to avoid having direct force applied thereto. It is therefore possible to maintain the shape of the low liquid permeation portion more easily. In addition, if the dimension of the low liquid permeation portion in the width direction were too large, the proportion of the low liquid permeation portion would be too high, which would lower drawability of the body fluid into the absorbent core and might cause leakage such as leakage following along another structure. However, because the dimension of the low liquid permeation portion in the width direction is equal to or smaller than 50% of the entire span of the absorbent core in the width direction, it is possible to ensure the drawability of the body fluid in the other regions and to thus prevent leakage.

According to a preferable aspect, the invention according to Aspect 15 may have the following features in the invention according to any one of Aspects 1 to 14. The low liquid permeation portion is provided in an excretion opening facing region positioned facing an excretion opening of a user. The excretion opening facing region is a portion to which the body fluid is guided most easily and which easily absorbs the body fluid. By providing the low liquid permeation portion in the excretion opening facing region, it is possible to maintain the rigidity of the location where the body fluid is absorbed most and where deformation easily occurs, in a state close to the state prior to use. It is therefore possible to effectively prevent twisting during the use.

According to a preferable aspect, the invention according to Aspect 16 may have the following features in the invention according to any one of Aspects 1 to 14. The low liquid permeation portion is provided so as not to straddle a center in the front-rear direction. Because the low liquid permeation portion is provided so as not to straddle the center, in the front-rear direction, of the absorbent article, it is possible to absorb body fluid at the center, in the front-rear direction, of the absorbent article where a relatively large amount of body fluid is discharged and to thus prevent discomfort that may be caused by a decrease in the liquid drawability. Further, in a mode in which the low liquid permeation portion is positioned on the front side relative to the center, in the front-rear direction, of the absorbent core, the user can easily be aware of the presence of the low liquid permeation portion, at the time of an attachment operation or during use.

According to a preferable aspect, the invention according to Aspect 17 may have the following features in the invention according to any one of Aspects 1 to 16. A testing member for testing a health condition of a user is positioned in a region overlapping with the low liquid permeation portion on the non-skin side relative to the low liquid permeation portion. According to the present aspect, by positioning the testing member in the region overlapping with the low liquid permeation portion, it is possible to avoid the situation where an excessive amount of body fluid comes into contact with the testing member and to thus inhibit erroneous reactions. In addition, in a configuration in which the low liquid permeation portion does not easily get twisted, it is possible to avoid having excessive pressure applied to the testing member, and it is therefore possible to avoid occurrence of creases in the testing member.

(2) absorbent article according to an embodiment

Hereinafter, an absorbent article according to an embodiment will be described with reference to drawings. It should be noted that, in the following description of the drawings, identical or similar portions will be given identical or similar reference signs. Here, the drawings are schematic views, and attention needs to be paid to the fact that the ratios between individual dimensions and the like are different from actual ones. Therefore, specific dimensions and the like need to be determined with reference to the following description. In addition, there may be a portion where the relationship or ratio between dimensions does not match between drawings. The absorbent article 1 may be an absorbent article such as a sanitary napkin, a panty liner, an incontinence pad, an excrement pad, a disposable diaper, an underwear-type napkin, or the like. The absorbent article 1 may be an article used while being attached to a wearable article such as underwear or may be an absorbent article used without being attached to a wearable article. The absorbent article 1 according to an embodiment is a panty liner. It should be noted that, in the following description of the drawings, identical or similar portions will be given identical or similar reference signs. Here, the drawings are schematic views, and attention needs to be paid to the fact that the ratios between individual dimensions and the like are different from actual ones. Therefore, specific dimensions and the like need to be determined with reference to the following description. In addition, there may be a portion where the relationship or ratio between dimensions does not match between drawings. In the cross-sectional views, although the constituent elements are indicated apart from one another for the sake of convenience in the explanations, the constituent elements in actual products are in contact with one another.

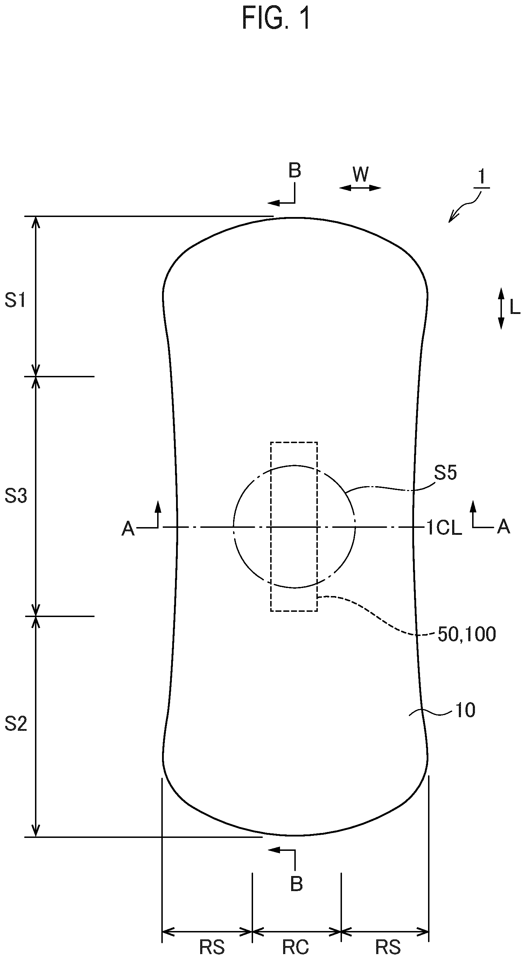

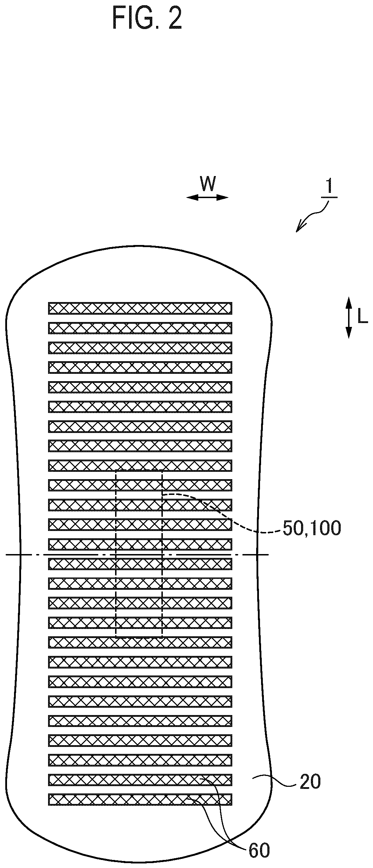

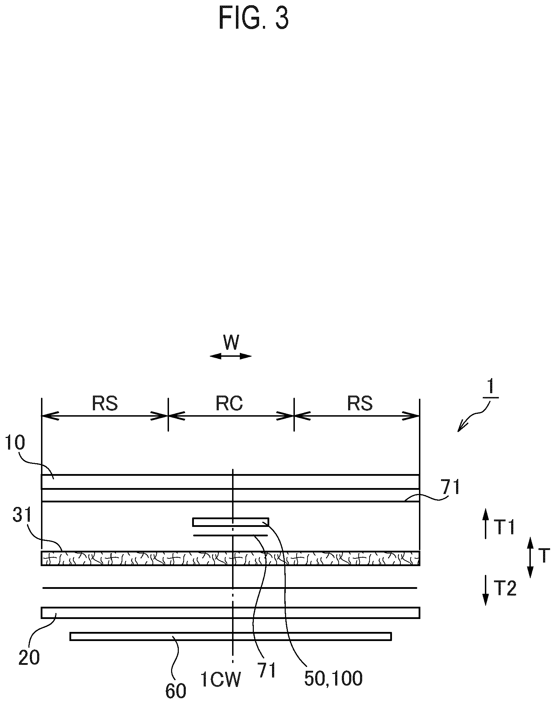

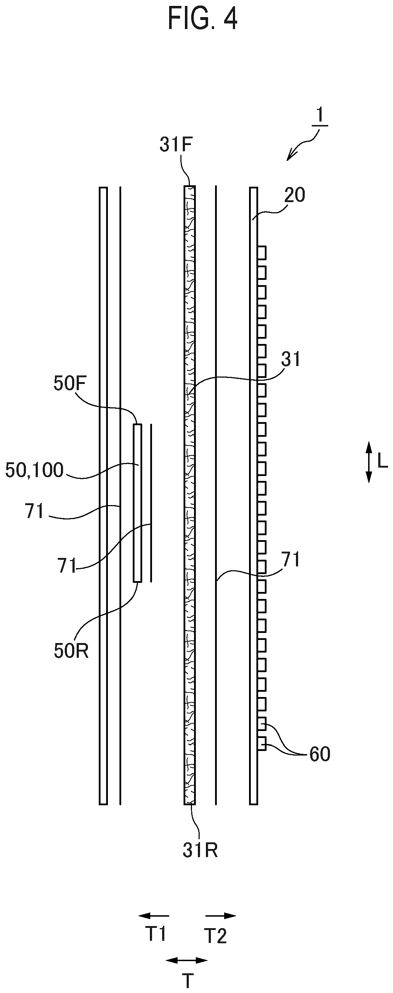

FIG. 1 is a plan view of an absorbent article according to an embodiment as seen from a skin side T1. FIG. 2 is a plan view of the absorbent article according to the embodiment as seen from a non-skin side T2. FIG. 3 is a cross-sectional view taken along the line A-A indicated in FIG. 1. FIG. 4 is a cross-sectional view taken along the line B-B indicated in FIG. 1. 1. Here, the "skin side" corresponds to a side facing the skin of the wearer during use. The "non-skin side" corresponds to a side opposite to the skin of the wearer during use. The absorbent article 1 has a front-rear direction L, a width direction W, and a thickness direction T that are orthogonal to each other. In the front-rear direction L of the absorbent article 1, a side that comes into contact with the lower abdomen of the wearer will be referred to as a "front side", and a side that comes into contact with the buttocks of the wearer will be referred to as a "rear side".

The absorbent article 1 has a front side region S1, a rear side region S2 ,and a crotch region S3. The crotch region S3 includes an excretion opening facing region S5 which is a region arranged to face the excretion opening of a wearer, for example, vaginal opening. When the absorbent article 1 is attached to a wearing article, the crotch region S3 is a region that is arranged in the crotch of the wearing article and is arranged between two legs of the wearer. The front side region S1 is positioned on the front side with respect to the crotch region S3.A front end edge of the front side region S1 defines a front end edge of the absorbent article 1. The rear side region S2 is positioned on the rear side with respect to the crotch region S3. A rear end edge of the rear side region S2 defines a rear end edge of the absorbent article 1. The crotch region S3 includes an excretion opening facing region S5 which faces the excretion opening of a wearer. In cases where the absorbent article 1 has the wing 7, the crotch region S3 may be a region where the wings are provided, or may be the region where the constricted portion is provided in an absorbent core having a configuration in which the absorbent core has a constricted portion on the inside in the width direction. The crotch region S3 may be a central region of three regions defined by dividing the absorbent article 1 into three equal parts in the front-rear direction L. The excretion opening facing region S5 is a region facing the excretory opening such as the vaginal opening and the urination opening, and straddles the center of the width direction W of the absorbent core 31. The excretion opening facing region S5 does not have to be disposed opposite the excretory opening of the wearer actually wears the absorbent article, and may be a region that is disposed opposite the excretory opening of the wearer in terms of design.

An outer portion as used herein refers to a part that extends over a certain range in the width direction W, including the outer edge in the width direction W, and the outer edge is the outer edge in the width direction W. An inner portion as used herein refers to a part that extends over a certain range in the width direction W, including the interior edge in the width direction W, and the inner edge is the interior edge in the width direction W. A front end and a rear end as used herein are each a part that extends over a certain range in the front-rear direction L, including the exterior edge in the front-rear direction L, and the outer end edge is the exterior edge in the front-rear direction L. An inner end is a part that extends over a certain range in the front-rear direction L, including the interior edge in the front-rear direction L, and the inner end edge is the interior edge in the front-rear direction L. The inner edge of a component that occupies a certain range in the front-rear direction is an edge that connects points located on the inside in the width direction of the component over the entire component. The front end and rear end in the present invention are parts that occupy a certain range in the front-rear direction L including edges in the front-rear direction L, and the front edge and rear edge are edges in the front-rear direction L. The outer end includes the front end and rear end, and the outer edge includes the front edge and rear edge. The inner side is a side that includes the inner edge and extends along the front-rear direction L. The outer side is a side that includes the outer edge and extends along the front-rear direction L. In this specification, the term "along the front-rear direction L" means a direction that has an angle of less than 45° with respect to the front-rear direction L, and the term "along the width direction W" means a direction that has an angle of less than 45° with respect to the width direction W.

The absorbent article 1 includes, at least, an absorbent core 31, a topsheet 10, and a backsheet 20. The absorbent article 1 has an oblong shape lengthwise. The topsheet 10 is a liquid-permeable sheet. The topsheet 10 may be configured so that, like in the present embodiment, the entire skin surface of the absorbent article 1 is covered by a single sheet or may be configured so as to include a center sheet covering a central part, in the width direction W, of the absorbent core 31 and side sheets that are positioned on the outside, in the width direction W, of the center sheet. The topsheet 10 is made of an arbitrary sheet-like material having a structure that permeates liquid, such as a non-woven fabric, a woven fabric, a porous plastic sheet, or a mesh sheet.

The backsheet 20 is a sheet that is positioned on the non-skin side T2 relative to the absorbent core 31 and is a liquid-impermeable sheet. The backsheet 20 is a liquid-impermeable sheet. As the backsheet 20, it is possible to use a polyethylene sheet, a laminated non-woven fabric mainly using polypropylene or the like, a breathable resin film, or a sheet obtained by joining a breathable resin film with a non-woven fabric such as a spunbond or a spunlace.

The absorbent core 31 is one component of an absorber. The absorbent core 31 may be made of absorptive material that absorbs liquid. The absorptive material that constitutes the absorbent core 31 can be formed from hydrophilic fiber, pulp, and superabsorbent polymer (SAP), for example. The absorber may include a core wrap sheet covering the absorbent core 31 or, as in the present embodiment, may not include a core wrap sheet. The absorbent core 31 has regions obtained by equally dividing the absorbent core 31 into three regions in the width direction W. The three equally-divided regions may include a central region RC positioned in a central part in the width direction W and side regions RS positioned outside, in the width direction W, of the central region RC.

As shown in FIG. 2 etc., the absorbent article 1 may include adhesion regions 60 used for fastening the absorbent article 1 onto a wearable article such as underwear. The adhesion regions 60 are regions provided on the non-skin side T2 of the backsheet 20 and are provided with fastening means for fastening the absorbent article 1 onto the wearable article. The adhesion regions 60 may each extend along the width direction W, while the plurality of adhesion regions 60 may be provided at intervals in the front-rear direction L. Alternatively, in a modification example, the adhesion regions 60 may each extend along the front-rear direction L, while the plurality of adhesion regions 60 may be provided at intervals in the width direction W.

The absorbent article 1 according to the present embodiment is configured so that it is possible to easily maintain absorbing capabilities of the absorbent core 31, while liquid is prevented from remaining on the topsheet 10. Configurations to easily maintain the absorbing capabilities of the absorbent core 31 while preventing liquid from remaining on the topsheet 10 roughly include a first mode and a second mode. In both the first mode and the second mode, the absorbent article 1 includes a low liquid permeation portion 100. The low liquid permeation portion 100 is a portion having a lower permeability speed for body fluid to permeate from the skin side T1 to the non-skin side T2 than the surroundings thereof, on the non-skin side T2 relative to the non-skin surface of the topsheet 10 and on the skin side T1 relative to the non-skin surface of the absorbent core 31. The low liquid permeation portion 100 is a portion having the lower permeability speed for the body fluid to permeate from the skin side T1 to the non-skin side T2 than regions (regions positioned adjacent in the width direction W and regions positioned adjacent in the front-rear direction L) in the surroundings thereof in a planar view.

Here, configurations of the low liquid permeation portion 100 can be roughly divided into a configuration in which absorption speed of the body fluid is lowered by having a lower drawability of the body fluid and a configuration in which absorption speed of the body fluid is lowered by making it difficult for the drawn body fluid to permeate to the non-skin side T2. Accordingly, examples of methods for specifying the low liquid permeation portion 100 include, for instance, a first specifying method and a second specifying method described below. The first specifying method is a method for specifying the low liquid permeation portion 100 having a lower drawability of the body fluid. The low liquid permeation portion 100 is provided in the space from the non-skin surface of the topsheet 10 to the non-skin surface of the absorbent core 31. For this reason, to specify the low liquid permeation portion 100, at first, the topsheet 10 is removed from the absorbent article 1, and also, a member (the backsheet 20 in the present embodiment) positioned on the non-skin side T2 relative to the non-skin surface of the absorbent core 31 is removed. A member (hereinafter, "subject member") positioned in the space from the non-skin surface of the topsheet 10 to the non-skin surface of the absorbent core 31 is taken out, and the subject member is disassembled into different materials as much as possible. Examples of the materials include the absorbent core 31, a core wrap sheet, a second sheet, other sheets (e.g., a resin film) besides these sheets, an adhesive agent, and a liquid repellent. When these materials are joined together, the disassembly is performed by deactivating a joined state of the adhesive agent or the like through a cooling or heating process at the time of the disassembly. Further, because components such as an adhesive agent and a liquid repellent that are applied to something else cannot be taken out alone, those components will each be in the state of being one of the materials. Each of the materials taken out of the subject member is placed on a flat surface, and one drop of a pseudo body fluid is dropped on the surface of the material on the skin side T1. When the pseudo body fluid remains on the material in a bead form for 60 seconds or longer, because the material does not easily draw the body fluid in, the material is determined to be the low liquid permeation portion 100. On the contrary, when the pseudo body fluid is drawn into the material in less than 60 seconds or remains on the material for 60 seconds or longer but the bead form is lost in less than 60 seconds, the material is determined not to be the low liquid permeation portion 100.

According to the second specifying method, the low liquid permeation portion 100 where it is difficult for the body fluid drawn therein to permeate to the non-skin side T2 is specified. At first, a subject member is taken out, similarly to the first specifying method. Unlike the first specifying method, according to the second specifying method, the subject member is specified while in the state of being the subject member, without being disassembled into materials. Whether it is difficult to permeate to the non-skin side T2 denotes whether or not the speed is lower than in the surroundings. Thus, the subject member is sectioned into regions. By using an ink (e.g., an oil-based ink) that does not get smudged by body fluids, the non-skin surface of the target member are marked with lines, so as to be sectioned into regions of 50 mm by 50 mm. Subsequently, the target member is positioned in a floating state so that the non-skin surface of the target member is visually recognizable. More specifically, multiple rope-like members (e.g., multiple resin strings) that do not draw body fluids therein are laid across a space at intervals, so that the target member is placed on the rope-like members. While the subject member is placed on the rope-like members, 5 ml of the pseudo body fluid is dropped onto the surface of the material on the skin side T1. The subject member is observed from the non-skin side T2. When body fluid arrival time of an arbitrary section is slower than that of the surrounding sections, because the arrival speed is slower than that of the surroundings, it is determined that a low liquid permeation portion 100 is provided in the arbitrary section. It is also acceptable to make comparison between sections to see if there is a difference in the arrival time thereof, so as to determine that a section having a longer period of time is provided with a low liquid permeation portion 100. In a preferable example, when the arrival time of a first section is compared with the arrival time of a second section, if one of the sections has the longer period of time with a ratio of 1.2 or larger to the shorter period of time, the section may be determined as being provided with a low liquid permeation portion 100. Alternatively, when the arrival time of the first section is compared with the arrival time of the second section, if one of the sections has a difference of 5 seconds or longer as compared to the shorter period of time, the section having the longer period of time may be determined as being provided with a low liquid permeation portion 100. Note that, when it is difficult, by using 5 ml of pseudo body fluid, to confirm whether or not the pseudo body fluid has reached the non-skin side T2, additional 5 ml of pseudo body fluid is dropped 60 seconds after the first drop. Further, when it is difficult to visually recognize the permeation state of the body fluid depending on the state of the subject member, it is also acceptable to make observation with the backsheet removed.

In the above examples, artificial menstrual blood may be used as the pseudo body fluid. The artificial menstrual blood to be used may be obtained by mixing the following, each in an appropriate amount: ionized water, glycerin, sodium carboxymethyl cellulose, sodium chloride, sodium hydrogen carbonate, red dye No. 102, red dye No. 2, and yellow dye No. 5 (The dyes are manufactured by Marubeni Syoukai). Further, viscosity of the pseudo body fluid may be approximately in the range of 22 mPa·s to 26 mPa·s. The viscosity may be measured by using a viscometer (Vismetron: Model VGA-4, manufactured by Shibaura Systems Co., Ltd.,).

As shown in FIG. 3 and FIG. 4, in the absorbent article 1 in the present embodiment, the following are arranged from the skin side T1 toward the non-skin side T2: the topsheet 10, an HMA (hot melt type) adhesive agent 71, a resin film 50, the HMA adhesive agent 71, the absorbent core 31, the HMA adhesive agent 71, the backsheet 20, and the adhesion regions 60. Thus, the subject member includes, at least the resin film 50, the HMA adhesive agent 71, and the absorbent core 31, although what is included may depend on how the HMA adhesive agent being in contact with the topsheet 10 and the backsheet 20 is positioned at the time of the disassembly. When the second specifying method is implemented in the subject member state, because the arrival speed of the section provided with the resin film 50 is lower, it is possible to determine that a low liquid permeation portion 100 is provided in the region where the resin film 50 is positioned. Alternatively, when the first specifying method is implemented, because the resin film 50 exhibits a low drawability of the body fluid, it is possible to determine that the resin film 50 constitutes a low liquid permeation portion 100.

The first mode is characterized in that the low liquid permeation portion 100 is positioned in the central region RC. The low liquid permeation portion 100 has a lower permeability speed for the body fluid to permeate to the non-skin side T2 than the surroundings thereof, so as to allow the body fluid to permeate less easily as compared to the surroundings thereof. The low liquid permeation portion 100 is positioned on the skin side T1 relative to the non-skin surface of the absorbent core 31. As for the absorbent core 31, to a certain part of the absorbent core 31 positioned on the non-skin side T2 relative to the low liquid permeation portion 100, the body fluid is guided less easily, which makes the absorption amount of body fluid smaller. Further, when a low liquid permeation portion 100 is provided on the non-skin surface of the absorbent core, it is possible to inhibit the absorption of the body fluid, in a certain range of the absorbent core 31 ranging from the non-skin surface toward the skin side T1. Usually, the rigidity and the thickness of the absorbent core 31 change when body fluid is absorbed. As compared to the absorbent core 31 before the absorption, the absorbent core 31 that has absorbed the body fluid might be unable to maintain the rigidity and the thickness thereof, and therefore might be unable to maintain a goal state until after use. However, by providing the low liquid permeation portion 100, it is possible to provide the absorbent core 31 with a portion where it is possible to prevent changes in the absorbent core 31 that may occur between before and after the body fluid absorption. In the central region RC of the absorbent core 31, because the body fluid is more easily discharged therein as compared to the side region RS, a larger change is to be caused by the body fluid absorption. Providing the central region RC with the low liquid permeation portion 100 makes it easier to maintain, even after use, the state prior to use. Further, it is possible to ensure drawability of the body fluid for the topsheet 10 and the like positioned on the skin side T1 relative to the low liquid permeation portion 100 and to thus prevent liquid from remaining on the surface of the absorbent article 1. Consequently, it is possible to prevent skin trouble that may be caused by the body fluid remaining on the surface of the absorbent article 1. The absorbent article 1 in the present embodiment has the configuration in the first mode, so that the resin film 50 constituting the low liquid permeation portion 100 is provided in the central region RC. Note that, in the first mode, it is sufficient when the low liquid permeation portion 100 is provided in at least a portion of the central region RC. The low liquid permeation portion 100 may be provided in a portion, in the width direction W, of the central region RC or may be provided along the entire span, in the width direction W, of the central region RC. Furthermore, the low liquid permeation portion 100 may be provided in both the central region RC and the side regions RS.

The second mode is characterized in that the low liquid permeation portion 100 is provided on the absorbent core 31, that the absorption amount per unit area in the region provided with the low liquid permeation portion 100 is lower than the absorption amount per unit area in the surrounding regions of the low liquid permeation portion 100, and that the rigidity of the region provided with the low liquid permeation portion 100 is higher than the rigidity of the surroundings regions of the low liquid permeation portion 100. Because the absorption amount per unit area of the region provided with the low liquid permeation portion 100 is lower, it is possible, by providing the low liquid permeation portion 100, to provide the absorbent core 31 with the part where it is possible to prevent the changes in the absorbent core 31 that may occur between before and after the body fluid absorption. In addition, because the rigidity of the region provided with the low liquid permeation portion 100 is higher than that of the surroundings, there is a lower possibility that deformation may be caused by pressure from the body during use, and the state prior to use can therefore be maintained more easily. Furthermore, because of being positioned on the non-skin side T2 relative to the topsheet 10, the low liquid permeation portion 100 does not easily touch the skin of the user directly. Because the rigidity of the topsheet 10 is lower than the rigidity of the low liquid permeation portion 100, even when the rigidity of the low liquid permeation portion 100 itself is high, the user does not easily feel high rigidity parts because of softness of the topsheet 10. It is therefore possible to prevent discomfort and skin trouble during use.

It is possible to measure an absorption capacity per unit area by using the following method. To begin with, the absorbent core 31 is taken out, sectioned into regions of 10 mm by 10 mm, and cut into the sections so as to prepare test pieces. The weight and the dimensions in a planar view of each of the test pieces are measured. The entirety of each of the test pieces is submerged in a pseudo body fluid. The pseudo body fluid may be the same as the one described above. After 60 seconds have elapsed, the test pieces are taken out and rested on a metal grid or the like for one minute. Subsequently, the weight of each of the test pieces is measured. By using this method, the absorption amount per unit area is specified. When the weights per area of absorbent fibers are not uniform in the test pieces, it is also acceptable to make comparison by using, as the absorption amount, an absorption ratio calculated as (weight after absorption - weight before absorption) / (weight before absorption).

Further, the rigidity may be Gurley stiffness and may be measured by using the following method, for example. The instrument to be used may be a Gurley's stiffness tester manufactured by Yasuda Seiki Ltd. In one procedure, the subject to be measured is cut in the size of 25 mm by 38 mm so that sampling satisfies N = 5. The samples are cut by using scissors, from a portion having no wrinkles. Subsequently, each sample is set on the instrument so that the upper end of the sample is positioned at the upper part end of a chuck. In that situation, a pendulum will swing over the lower end of the sample. A supplementary weight is attached so as to satisfy the range between 3 to 6 on the graduation. A switch is pressed so as to read the graduation at the instant when a rotation rod of the pendulum leaves the sample. A measuring process is performed for the left and for the right of the instrument. Three specimens are measured. According to a calculation method, an average value of the left and the right is calculated with respect to each of the lengthwise and the widthwise directions. In the present embodiment, the calculation was performed by using the formula presented below The positions where the weights were applied are expressed as i for a one-inch mark, ii for a two-inch mark, and iii for a four-inch mark.

[Mathematical Formula 1]

$$\text{BENDING RESISTANCE} = \frac{\text{GRADUATION READING} \times \text{i} \times 1 \text{ or ii} \times 2 \text{ or iii} \times 4}{\text{AVERAGE VALUE} \qquad 5} \times 9.88$$

An example of a specific configuration in the second mode can be explained as follows: A low weight-per-area portion (including a slit) is formed in the absorbent core 31 so that a different material (a resin film) is positioned in a region overlapping with the low weight-per-area portion or so that a high rigidity part is formed through an embossing process or the like in the material in the region overlapping with the low weight-per-area portion. The low weight-per-area portion and the different material, or the low weight-per-area portion and the high rigidity part constitute the low liquid permeation portion 100. Accordingly as for the low liquid permeation portion 100 of the absorbent article in the second mode, it is acceptable as long as at least a portion thereof is provided in the absorbent core 31, and there may a portion thereof that is not provided in the absorbent core 31. Alternatively, in an example, the absorbent core 31 may be impregnated with a liquid repellent (e.g., silicone). This configuration also increases rigidity of the impregnated region, while keeping the liquid absorption amount low

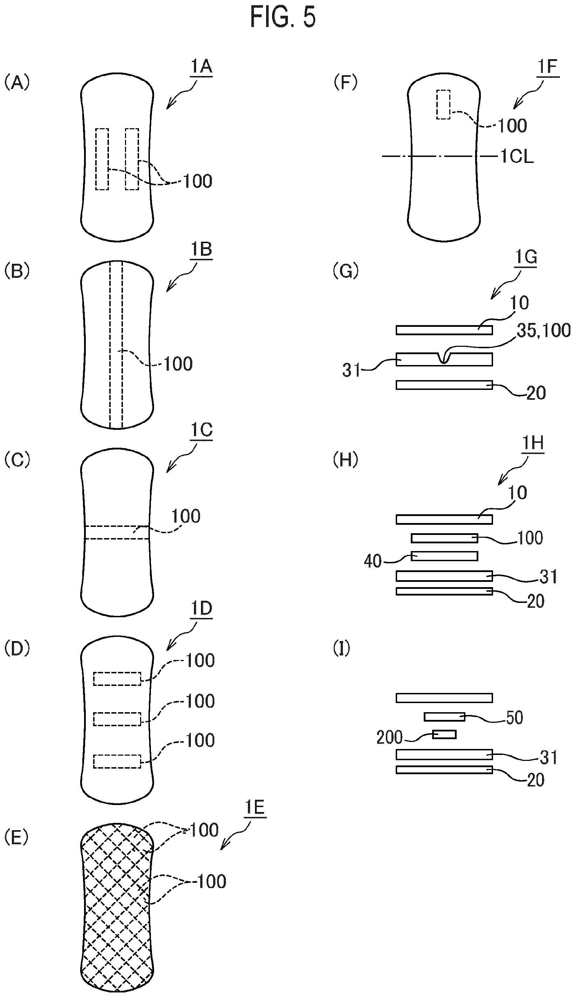

Next, configurations that may preferably be adopted in the first mode and the second mode will be explained. Unless noted otherwise, the configurations described below are also applicable to the first mode and the second mode. Further the configuration and the position of the low liquid permeation portion 100 may be changed as appropriate. FIG. 5 shows an absorbent article according to a modification example. The absorbent article according to the modification example may be a modification example applicable to either of the first and the second modes.

Similarly to the absorbent article in the second mode, in the absorbent article in the first mode, the rigidity of the region provided with the low liquid permeation portion 100 may be higher than the rigidity of the surrounding regions of the low liquid permeation portion 100. According to the present aspect, there is a lower possibility that deformation may be caused by pressure from the body during use, and the state prior to use can therefore be maintained more easily. Further, because the user does not easily feel high rigidity parts due to softness of the topsheet 10, it is possible to prevent discomfort during use. Further similarly to the absorbent article in the first mode, also in the absorbent article in the second mode, the low liquid permeationportion 100 may be placed in the central region RC. Providing the low liquid permeation portion 100 in the central region RC makes it easier to maintain, even after use, the state prior to use.

As shown in FIG. 1, the low liquid permeation portion 100 may straddle a center 1CW, in the width direction W, of the absorbent core 31. The center, in the width direction W, of the absorbent core 31 is a portion where force gets easily concentrated, when being interposed between the legs or the like. In addition, the center, in the width direction W, of the absorbent core 31 is a region where body fluid is easily discharged. By providing the low liquid permeation portion 100 so as to straddle the center, in the width direction W, of the absorbent core 31, it is possible to prevent deformation caused by such force and also to prevent the changes in the rigidity and the like that may be caused by the absorption of the body fluid. Note that, in a mode in which two or more low liquid permeation portions 100 are provided, it is sufficient when one of the low liquid permeation portions 100 straddles the center 1CW, in the width direction W, of the absorbent core 31. Further, as shown in FIG. 5(A), in a modification example, the low liquid permeation portion 100 may be provided so as not to straddle the center 1CW, in the width direction W, of the absorbent core 31. Because it is possible to absorb the body fluid in the center, in the width direction W, of the absorbent article 1 where a relatively large amount of body fluid is discharged, it is possible to prevent discomfort that may be caused by a decrease in the liquid drawability.

As shown in FIG. 1, the low liquid permeation portion 100 may extend along the front-rear direction L. In other words, the low liquid permeation portion 100 may be oblong lengthwise. With the low liquid permeation portion 100, it is possible to spread the body fluid along the front-rear direction L and to absorb the body fluid in a large region extending along the front-rear direction L. By having the larger range for absorbing the body fluid, it is possible to avoid locally forming a region where a large amount of body fluid is held and to easily maintain the rigidity and the like of the absorbent core 31 even after the absorption of the body fluid. Further, as shown in FIG. 5(A), a plurality low liquid permeation portions 100 may be provided in an absorbent article 1A so as to each extend along the front-rear direction L while being positioned at intervals in the width direction W. In this configuration, the low liquid permeation portions 100 are positioned at intervals in the width direction W, so that a region having no low liquid permeation portion 100 is present between the low liquid permeation portions 100. Thus, even when the low liquid permeation portion 100 positioned on the outer side in the width direction W is deformed due to force being applied by the legs or the like inwardly in the width direction W, the low liquid permeation portion 100 positioned on the inner side in the width direction W does not get deformed and thus exerts capabilities thereof. As a result, one of the low liquid permeation portions 100 is able to maintain the capabilities of the low liquid permeation portions 100. Further, while body fluid is being drawn to the non-skin side T2 in the region positioned between the low liquid permeation portions 100, the low liquid permeation portions 100 are able to spread the body fluid in the front-rear direction L, and it is therefore possible to absorb the body fluid in a large region extending along the front-rear direction L. As shown in FIG. 5(B), the low liquid permeation portion 100 may be provided in an absorbent article 1B, uninterruptedly over the entire span along the front-rear direction L of the absorbent core 31. With this configuration, throughout the entire span along the front-rear direction L of the absorbent core 31, it is possible to uninterruptedly provide the region where it is possible to maintain the state of the absorbent core 31 prior to use.

The low liquid permeation portion 100 in the present embodiment is not provided in the entirety of the absorbent core 31 and is provided in a certain range from a center 1CL, in the front-rear direction L, of the absorbent article 1. As shown in FIG. 4, a front end edge 100F of the low liquid permeation portion 100 is positioned on the rear side relative to a front end edge 31F of the absorbent core 31, whereas a rear end edge 100R of the low liquid permeation portion 100 is positioned on the front side relative to a rear end edge 31R of the absorbent core 31. With this configuration, in the region positioned on the outer side of the low liquid permeation portion 100 in the front-rear direction L, it is possible to draw in and absorb the body fluid. Further, by providing the low liquid permeation portion 100 in the region where a relatively large amount of body fluid is discharged and not providing the low liquid permeation portion 100 on the outer side thereof in the front-rear direction L, it is possible to spread the body fluid to the outer side in the front-rear direction L via the low liquid permeation portion 100 and to thus hold the body fluid in a large range of the absorbent core 31. Consequently, because the absorbent core 31 does not locally absorb the body fluid, it is possible to absorb the body fluid throughout the entirety of the absorbent core 31 and to prevent deformation and twisting throughout the entirety of the absorbent core 31 after the absorption of the body fluid. Further, the low liquid permeation portion 100 may be positioned so as to straddle a plurality of adhesion regions 60. In other words, there may be a region overlapping with a low liquid permeation portion 100 in the thickness direction T of the adhesion regions 60; a region provided with no adhesion region 60 but provided with a low liquid permeation portion 100; and a region provided with an adhesion region 60 but provided with no low liquid permeation portion 100.

Preferably, the adhesion region 60 may be positioned so as to straddle the outer edge of a low liquid permeation portion 100. In a mode in which the low liquid permeation portion 100 has high rigidity, discomfort may be caused by the outer edge (a boundary) of the low liquid permeation portion 100 abutting against skin. However, by arranging the adhesion region so as to straddle the outer edge (the boundary) of the low liquid permeation portion 100, it is possible to prevent discomfort on the skin, by fastening the outer edge of the low liquid permeation portion 100 to a worn article by using the adhesion region so that the worn article is in close contact with the outer edge of the low liquid permeation portion 100. More preferably, the adhesion region 60 may straddle an outside edge, in the width direction, of the low liquid permeation portion 100, while straddling an outer end edge, in the front-rear direction, of the low liquid permeation portion 100. It is possible to prevent discomfort over the entire outer edge of the low liquid permeation portion 100. In the configuration in which the outer edge of the low liquid permeation portion 100 is straddled, it is not necessary to straddle the entire outer edge, and it is acceptable when at least a portion thereof is straddled.

As shown in FIG. 5(C), in another modification example, the low liquid permeation portion 100 in an absorbent article 1C may extend along the width direction W. In other words, the low liquid permeation portion 100 may be oblong widthwise. With this configuration, the low liquid permeation portion 100 is able to spread body fluid in the width direction W, and it is therefore possible to absorb the body fluid in a large region extending along the width direction W. By having the larger range for absorbing the body fluid, it is possible to avoid locally forming a region where a large amount of body fluid is held and to thus easily maintain the rigidity and the like of the absorbent core 31 after the absorption of the body fluid. As shown in FIG. 5(C), in the modification example, the low liquid permeation portion 100 in the absorbent article 1C may extend along the width direction W, while being provided uninterruptedly over the entire span along the width direction W of the absorbent core 31. With this configuration, throughout the entire span along the width direction W of the absorbent core 31, it is possible to uninterruptedly provide the region where it is possible to maintain the state of the absorbent core 31 prior to use. As shown in FIG. 5(D), in yet another modification example, the low liquid permeation portions 100 in an absorbent article 1D may each extend along the width direction W, while each being positioned in a partial region, in the width direction W, of the absorbent core 31. By providing the low liquid permeation portions 100 in the region where a relatively large amount of body fluid is discharged and not providing any low liquid permeation portion 100 on the outer side thereof in the width direction W, it is possible to spread the body fluid to the outer side in the width direction W via the low liquid permeation portions 100 and to thus hold the body fluid in a large range of the absorbent core 31.

As shown in FIG. 5(D), the plurality low liquid permeation portions 100 in the absorbent article 1D may each extend along the width direction W, while being provided at intervals in the front-rear direction L. In this configuration, the low liquid permeation portions 100 are positioned at intervals in the front-rear direction L so that regions having no low liquid permeation portion 100 are present between the low liquid permeation portions 100. When the absorbent article 1 is positioned along a curve of the body from the front side to the rear side of the body, the low liquid permeation portions 100 are able to exert capabilities thereof without being deformed, and at least one of the low liquid permeation portions 100 is able to maintain the capabilities of the low liquid permeation portion 100. Further, while body fluid is being drawn to the non-skin side T2 in the region positioned between the low liquid permeation portions 100, the low liquid permeation portions 100 are able to spread the body fluid in the width direction W, and it is therefore possible to absorb the body fluid in a large region extending along the width direction W. In addition, in a mode in which the low liquid permeation portions 100 have high rigidity, it is possible to prevent the low liquid permeation portions 100 from being deformed when being interposed between the legs and to thus better maintain the shape of the absorbent core 31 in the state prior to use. The pitch between the low liquid permeation portions 100 in the front-rear direction L may be different from the pitch between the adhesion regions 60 in the front-rear direction L. In other words, there may be a region overlapping with a low liquid permeation portion 100 in the thickness direction T of the adhesion regions 60; a region provided with no adhesion region 60 but provided with a low liquid permeation portion 100; and a region provided with an adhesion region 60 but provided with no low liquid permeation portion 100.

As shown in FIG. 5(E), in yet another modification example, the low liquid permeation portions 100 in an absorbent article 1E may be positioned in a grid formation. In this configuration, the regions provided with the low liquid permeation portions 100 and the regions provided with no low liquid permeation portion 100 each extend along the front-rear direction L and the width direction W. Thus, it is possible to spread body fluid throughout a large range in the front-rear direction L and the width direction W and also to ensure drawability of the body fluid in the regions provided with no low liquid permeation portion 100.

Further, the low liquid permeation portion 100 may be provided in an excretion opening facing region S5 positioned facing an excretion opening of the user. In a mode in which, like in the present embodiment, the absorbent article is in line symmetry with respect to a line that passes through the center of the front-rear direction L and extends along the width direction W, it is assumed that the excretion opening facing region S5 is positioned at the center, in the front-rear direction L, of the absorbent article 1. In other words, the low liquid permeation portion 100 may straddle the center 1CL, in the front-rear direction L, of the absorbent article 1. The excretion opening facing region S5 is a portion to which the body fluid is guided most easily and which easily absorbs the body fluid. By providing the low liquid permeation portion 100 in the excretion opening facing region S5, it is possible to maintain the rigidity of the location where the body fluid is absorbed most and where deformation easily occurs, in a state close to the state prior to use. It is therefore possible to effectively prevent twisting during the use.

As shown in FIG. 5(F), in yet another modification example, the low liquid permeation portion 100 in an absorbent article 1F may be positioned so as not to straddle the center in the front-rear direction L. In this configuration, it is possible to absorb body fluid at the center 1CL, in the front-rear direction L, of the absorbent article 1 where a relatively large amount of body fluid is discharged and to thus prevent discomfort that may be caused by a decrease in the liquid drawability. Further, as shown in FIG. 5(F), in a mode in which the low liquid permeation portion 100 of the absorbent article 1F is positioned on the front side relative to the center, in the front-rear direction, of the absorbent core 31, the user can easily be aware of the presence of the low liquid permeation portion 100 at the time of an attachment operation or during use.

The length of the low liquid permeation portion 100 in the front-rear direction L may be equal to or shorter than 50% of the length of the absorbent article 1 in the front-rear direction L. While having the length equal to or shorter than 50% of the length of the absorbent article 1 in the front-rear direction L, the low liquid permeation portion 100 accounts for a small percentage of the entirety of the absorbent core 31 in the front-rear direction. While in use, the cross-sectional shape of the absorbent article 1 along the front-rear direction L is a curved shape fitted along the shape of the body. By keeping the length of the low liquid permeation portion 100 in the front-rear direction L relatively short, it is possible to easily cause the entire absorbent core 31 to be fitted along the curve of the body, while preventing deformation of the low liquid permeation portion 100. Further, preferably, the length of the low liquid permeation portion 100 in the front-rear direction L may be equal to or shorter than 40% of the length of the absorbent article 1 in the front-rear direction L and may be more preferably equal to or shorter than 30% of the same.

In order to be positioned along the curve of the body more easily, the length of the low liquid permeation portion 100 in the front-rear direction L may be equal to or shorter than 90 mm, and may be preferably equal to or shorter than 80 mm. Further, in order to prevent front leakage and rear leakage of body fluid, the distance between the outer end edge of the low liquid permeation portion 100 and the outer end edge of the absorbent core 31 may be equal to or longer than 20 mm, may be preferably equal to or longer than 30 mm, and may be more preferably equal to or longer than 40 mm. Further, in order to ensure the capability of the low liquid permeation portion 100 to maintain the shape of the absorbent core 31, the length of the low liquid permeation portion 100 in the front-rear direction L may be equal to or longer than 30 mm, may be preferably equal to or longer than 40 mm, and may be more preferably equal to or longer than 50 mm.

The dimension of the low liquid permeation portion 100 in the width direction W may be equal to or smaller than 50% of the dimension of the absorbent core 31 in the width direction W. Because the dimension of the low liquid permeation portion 100 in the width direction W is equal to or smaller than 50% of the entire span of the absorbent core 31 in the width direction W, the low liquid permeation portion 100 does not easily touch the legs or the like of the user and is thus able to avoid having direct force applied thereto. It is therefore possible to maintain the low liquid permeation portion 100 and to reduce twisting of the absorbent core 31. In addition, if the dimension of the low liquid permeation portion 100 in the width direction W were too large, the proportion of the low liquid permeation portion 100 would be too high, which would lower the drawability of body fluid into the absorbent core 31 and might cause leakage such as leakage following along another structure. However, because the dimension of the low liquid permeation portion 100 in the width direction W is equal to or smaller than 50% of the entire span of the absorbent core 31 in the width direction W, it is possible to ensure the drawability of the body fluid in the other regions and to thus prevent leakage. Further, preferably, the dimension of the low liquid permeation portion 100 in the width direction W may be equal to or smaller than 40% of the dimension of the absorbent core 31 in the width direction W, and may be more preferably equal to or smaller than 30% of the dimension of the absorbent core 31 in the width direction W.

In order to prevent discomfort caused on the excretion opening, the dimension of the low liquid permeation portion 100 in the width direction W may be equal to or smaller than 40 mm, may be preferably equal to or smaller than 30 mm, and may be more preferably equal to or smaller than 20 mm. In order to prevent discomfort caused on the legs, the dimension of the low liquid permeation portion 100 in the width direction W may be equal to or smaller than 60 mm, may be preferably equal to or smaller than 40 mm, and may be more preferably equal to or smaller than 30 mm. Further, in order to prevent discomfort caused on the legs, the distance between the outside edge of the low liquid permeation portion 100 and the outside edge of the absorbent article may be equal to or longer than 20 mm, may be preferably equal to or longer than 30 mm, and may be more preferably equal to or longer than 40 mm. Further, in order to prevent sideway leakage of the body fluid, the distance between the outside edge of the low liquid permeation portion 100 and the outside edge of the absorbent core 31 may be equal to or longer than 20 mm, may be preferably equal to or longer than 30 mm, and may be more preferably equal to or longer than 40 mm. Furthermore, in order to ensure the capability of the low liquid permeation portion 100 to maintain the shape of the absorbent core 31, the dimension of the low liquid permeation portion 100 in the width direction W may be equal to or larger than 5 mm, may be preferably equal to or larger than 10 mm, and may be more preferably equal to or larger than 15 mm.

The low liquid permeation portion 100 may be made of at least one of the materials provided in the subject member. The low liquid permeation portion 100 may be provided on one material or may be provided on two or more materials. As long as the low liquid permeation portion 100 is provided between the non-skin surface of the topsheet 10 and the non-skin surface of the absorbent core 31, the position in the thickness direction T within that range is not limited. Between the non-skin surface of the topsheet 10 and the non-skin surface of the absorbent core 31, the low liquid permeation portion 100 may be provided uninterruptedly in the thickness direction T or may be provided partially in the thickness direction T.

As shown in FIG. 3 and FIG. 4, the low liquid permeation portion 100 may be provided between the topsheet 10 and the absorbent core 31. In this configuration, it is possible to prevent body fluid absorption of the absorbent core 31 in at least the region overlapping with the low liquid permeation portion 100. In addition, because the low liquid permeation portion 100 and the absorbent core 31 are provided separately from each other, it is possible to easily ensure the absorbing capabilities of the absorbent core 31. Specific examples of the low liquid permeation portion 100 provided between the topsheet 10 and the absorbent core 31 include, for instance, a second sheet, a core wrap sheet, the resin film of the present embodiment, an adhesive agent, and a liquid repellent. Further, it is also acceptable to use, as the low liquid permeation portion 100, a material obtained by applying a liquid repellent to a non-woven fabric including hydrophilic fibers or a non-woven fabric which includes hydrophilic fibers and a portion of which is made into a film through a fusion-bonding process or the like.

In yet another modification example, as shown in FIG. 5(G), the low liquid permeation portion 100 in an absorbent article 1G may be provided in the absorbent core 31. According to the present aspect, when a portion of the absorbent core 31 is the low liquid permeation portion 100, because body fluid is not easily absorbed by the absorbent core 31, it is possible to maintain rigidity of the absorbent core 31. Further, it is possible to ensure drawability of the body fluid for the topsheet 10 and the like positioned on the skin side T1 relative to the low liquid permeation portion 100 and to thus prevent liquid from remaining on the surface of the absorbent article 1. Consequently, it is possible to prevent skin trouble that may be caused by the body fluid remaining on the surface of the absorbent article 1.

In yet another modification example, as shown in FIG. 5(H), an absorbent article 1H may include a second sheet 40 positioned between the topsheet 10 and the absorbent core 31, while the low liquid permeation portion 100 may be provided between the topsheet 10 and the second sheet. According to the present aspect, it is possible to provide the low liquid permeation portion 100 in the position apart from the absorbent core 31. It is therefore possible to lower the possibility of the absorbent core 31 absorbing body fluid and to thus further decrease the changes in the rigidity of the absorbent core 31 that may occur between before and after the body fluid absorption.

Similar to the present embodiment, the low liquid permeation portion 100 may be made of a liquid-impermeable sheet positioned on the skin side T1 relative to the absorbent core 31. An example of the liquid-impermeable sheet is the resin film 50 of the present embodiment, for instance. Other possible configurations include cellophane. With these configurations, the liquid-impermeable sheet prevents the body fluid from being absorbed from the skin side. It is therefore possible to ensure the rigidity of the absorbent core 31 in the region overlapping with the sheet.

Further, the low liquid permeation portion 100 may be made of a hydrophobic coating agent applied to at least one of the absorbent core 31, the topsheet 10, and a sheet positioned between the absorbent core 31 and the topsheet 10. With this configuration, the hydrophobic coating agent prevents the body fluid from being absorbed from the skin side, and it is possible to ensure the rigidity of the absorbent core 31 in the region overlapping with the adhesive agent. Examples of the hydrophobic coating agent include an adhesive agent (an HMA adhesive agent) and a liquid repellent (silicone), for instance. Examples of the modes in which a coating agent constitutes the low liquid permeation portion 100 include: a configuration being thicker than the surroundings and than a coating region of the coating agent (so that impregnation reaches the inside of the material); a configuration in which an area ratio of the coating agent per unit area is higher than that in the surroundings (the surrounding region uses a discontinuous application process such as spiral, whereas the region corresponding to the low liquid permeation portion 100 uses non-discontinuous application process); and a configuration in which the coating agent is applied only to the region constituting the low liquid permeation portion 100, while no coating agent is applied to the surroundings.

As shown in FIG. 5(G), in the modification example, the low liquid permeation portion 100 in the absorbent article 1G may be made of a compressed part 35 obtained by compressing at least the absorbent core 31 in the thickness direction. The compressed part 35 may be obtained by compressing only the absorbent core 31 or by compressing the absorbent core 31 together with another sheet (e.g., a core wrap sheet or a second sheet). Because the low liquid permeation portion 100 including the compressed part 35 has high rigidity, it is possible to further ensure the rigidity of the absorbent core 31. More preferably, at the time of forming the compressed part 35, silicone may be applied to the location where the compressed part 35 is to be provided, so that the silicone is transferred to the compressed part. With this configuration, it is possible to further lower the migration speed of the body fluid in the low liquid permeation portion 100. Further, in another preferable mode, when a material (e.g., the absorbent core 31) forming the compressed part 35 contains thermoplastic resin, it is also acceptable to melt the thermoplastic resin at the time of forming the compressed part 35. With this configuration, it is possible to further lower the migration speed of the body fluid in the low liquid permeation portion 100.

As shown in FIG. 5(I), a testing member 200 for testing a health condition of the user may be positioned in a region overlapping with the low liquid permeation portion 100 on the non-skin side T2 relative to the low liquid permeation portion 100. In the mode shown in FIG. 5(I), the resin film 50 constituting the low liquid permeation portion 100 is positioned on the skin side T1 of the testing member 200. By positioning the testing member 200 in the region overlapping with the low liquid permeation portion 100, it is possible to avoid the situation where an excessive amount of body fluid comes into contact with the testing member 200 and to thus inhibit erroneous reactions. In addition, in a configuration in which the low liquid permeation portion 100 does not easily get twisted, it is possible to avoid having excessive pressure applied to the testing member 200, and it is therefore possible to avoid occurrence of creases in the testing member 200. The testing member may be, for example, a member that displays (a color indicating) the health condition of the user by using an indication drug.

For example, it is possible to use the testing member disclosed in Japanese Patent Laid-Open No. 2020-022585. In the present example, it is sufficient when at least a portion of the testing member 200 is covered by the low liquid permeation portion 100. Preferably, the entirety of the testing member 200 may be covered by the low liquid permeation portion 100.

Hitherto, while the invention has been described using the above-described embodiments, it is clear to a person skilled in the art that the invention is not limited to the embodiments in the specification. Changes and modifications of the invention may be made without departing from the spirit and scope of the invention defined in claims. Therefore, the description of this specification is given as merely exemplary, but not intended to restrict the invention.

The entire contents of Japanese Patent Application No. 2022-120400 (filed on July 28, 2022) is incorporated into the present specification by reference.

[INDUSTRIAL APPLICABILITY]

According to the present invention, an absorbent article in which it is possible to easily maintain absorbing capabilities of an absorbent core, while liquid is prevented from remaining on the topsheet is provided.

[REFERENCE SIGNS LIST]

1: absorbent article
10: topsheet
20: backsheet
31: absorbent core
35: compressed part
50: resin film (liquid-impermeable sheet)
60: adhesion region
100: low liquid permeation portion
200: testing member
L: front-rear direction
RC: central region
S5: excretion opening facing region
T: thickness direction
T1: skin side
T2: non-skin side
W: width direction

Claims

1. An absorbent article comprising: a liquid-permeable topsheet, a liquid-impermeable backsheet, and an absorbent core positioned between the topsheet and the backsheet, wherein

a low liquid permeation portion having a lower permeability speed for body fluid to permeate from a skin side to a non-skin side than surroundings thereof is provided on a non-skin side relative to a non-skin surface of the topsheet and on a skin side relative to a non-skin surface of the absorbent core, and
the low liquid permeation portion is placed in a central region positioned in a central part in a width direction among regions obtained by equally dividing the absorbent core into three regions in the width direction.

2. The absorbent article according to claim 1, wherein
rigidity of the region provided with the low liquid permeation portion is higher than rigidity of regions in the surroundings of the low liquid permeation portion.

3. An absorbent article comprising: a liquid-permeable topsheet, a liquid-impermeable backsheet, and an absorbent core positioned between the topsheet and the backsheet, wherein

a low liquid permeation portion having a lower permeability speed for body fluid to permeate from a skin side to a non-skin side than surroundings thereof is provided on a non-skin side relative to a non-skin surface of the topsheet and on a skin side relative to a non-skin surface of the absorbent core,

the low liquid permeation portion is provided on the absorbent core,
an absorption amount per unit area of the absorbent core provided with the low liquid permeation portion is smaller than an absorption amount per unit area of the absorbent core positioned in the surroundings of the low liquid permeation portion, and
rigidity of a region provided with the low liquid permeation portion is higher than rigidity of a region in the surroundings of the low liquid permeation portion.

4. The absorbent article according to any one of claims 1 to 3, wherein
the low liquid permeation portion is made of a liquid-impermeable sheet positioned on the skin side relative to the absorbent core.

5. The absorbent article according to any one of claims 1 to 3, wherein
the low liquid permeation portion is made of a hydrophobic coating agent applied to at least one of the absorbent core, the topsheet, and a sheet positioned between the absorbent core and the topsheet.

6. The absorbent article according to any one of claims 1 to 3, wherein
the low liquid permeation portion is made of a compressed part obtained by compressing at least the absorbent core in a thickness direction.

7. The absorbent article according to any one of claims 1 to 3, wherein
the low liquid permeation portion is provided between the topsheet and the absorbent core.

8. The absorbent article according to claim 1 or 2, wherein
the low liquid permeation portion is provided on the absorbent core.

9. The absorbent article according to any one of claims 1 to 3, comprising:
a second sheet positioned between the topsheet and the absorbent core, wherein the low liquid permeation portion is provided between the topsheet and the second sheet.

10. The absorbent article according to any one of claims 1 to 3, wherein
the low liquid permeation portion straddles a center, in the width direction, of the absorbent core.

11. The absorbent article according to any one of claims 1 to 3, wherein
the low liquid permeation portion extends along a front-rear direction.

12. The absorbent article according to claim 11, wherein
a plurality of low liquid permeation portion are provided at intervals in the width direction.

13. The absorbent article according to any one of claims 1 to 3, wherein
a plurality of low liquid permeation portion extends along the width direction and are provided at intervals in a front-rear direction.

14. The absorbent article according to claim 13, wherein
a dimension of the low liquid permeation portion in the width direction is equal to or smaller than 50% of a dimension of the absorbent core in the width direction.

15. The absorbent article according to any one of claims 1 to 3, wherein
the low liquid permeation portion is provided in an excretion opening facing region positioned facing an excretion opening of a user.

16. The absorbent article according to any one of claims 1 to 3, wherein
the low liquid permeation portion is provided so as not to straddle a center in the front-rear direction.

17. The absorbent article according to any one of claims 1 to 3, wherein
a testing member for testing a health condition of a user is positioned in a region overlapping with the low liquid permeation portion on the non-skin side relative to the low liquid permeation portion.

# FIG. 1

# FIG. 2

# FIG. 3

# FIG. 4

# FIG. 5

(A)

1A

100

(B)

1B

100

(C)

1C

100

(D)

1D

100

100

100

(E)

1E

100

100

(F)

1F

100

1CL

(G)

1G

10

35,100

31

20

(H)

1H

10

100

40

31

20

(I)

50

200

31

20

## EP 4 541 332 A1

<div style="text-align:center">

**INTERNATIONAL SEARCH REPORT**

</div>

| International application No. |
|---|
| **PCT/JP2023/015043** |

**A. CLASSIFICATION OF SUBJECT MATTER**

*A61F 13/42*(2006.01)i; *A61F 13/511*(2006.01)i; *A61F 13/537*(2006.01)i
FI: A61F13/537 100; A61F13/511 200; A61F13/511 400; A61F13/537 310; A61F13/537 400; A61F13/42 Z

According to International Patent Classification (IPC) or to both national classification and IPC

**B. FIELDS SEARCHED**

Minimum documentation searched (classification system followed by classification symbols)

A61F13/42; A61F13/511; A61F13/537

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-22585 A (UNI-CHARM CO., LTD.) 13 February 2020 (2020-02-13) paragraphs [0006]-[0112], fig. 1-7 | 1-17 |
| A | WO 2021/132724 A1 (UNI-CHARM CO., LTD.) 01 July 2021 (2021-07-01) paragraphs [0046]-[0092], fig. 1-14 | 1-17 |
| A | JP 2017-64110 A (UNI-CHARM CO., LTD.) 06 April 2017 (2017-04-06) paragraphs [0022]-[0061], fig. 1-10 | 1-17 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **16 June 2023** | **27 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/015043**

| Patent document cited in search report | | | Publication date (day/month/year) | Patent family member(s) | | | Publication date (day/month/year) |
|---|---|---|---|---|---|---|---|
| JP | 2020-22585 | A | 13 February 2020 | CN | 112512471 | A | |
| WO | 2021/132724 | A1 | 01 July 2021 | CN | 114845674 | A | |
| JP | 2017-64110 | A | 06 April 2017 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017099558 A
- JP 2020022585 A
- JP 2022120400 A